# EUROPÄISCHE PATENTSCHRIFT

(11) **EP 3 272 361 B1**
(45) Veröffentlichungstag und Bekanntmachung des Hinweises auf die Patenterteilung: **20.11.2019**
(21) Anmeldenummer: 17188743.3
(22) Anmeldetag: 22.10.2013
(51) Int. Cl.: A61K 9/19, A61K 47/10, A61K 9/08, A61K 9/16, A61K 31/00, A61K 9/00, A61K 47/26, A61K 31/407, A61K 47/18

(54) **VERFAHREN ZUR HERSTELLUNG EINER GEFRIERGETROCKNETEN PHARMAZEUTISCHEN ZUSAMMENSETZUNG MIT GEHALT AN MITOMYCIN C**
METHOD FOR PRODUCING A FREEZE-DRIED PHARMACEUTICAL COMPOSITION CONTAINING MITOMYCIN C
PROCÉDÉ DE FABRICATION D'UNE COMPOSITION PHARMACEUTIQUE LYOPHILISÉE CONTENANT DE LA MITOMYCINE C

(43) Veröffentlichungstag der Anmeldung: 24.01.2018
(62) Teilanmeldung aus: 13189775.3
(73) Patentinhaber: medac Gesellschaft für klinische Spezialpräparate mbH, 22880 Wedel (DE)
(72) Erfinder: Schuldt-Lieb, Sonja, 20257 Hamburg (DE); Bialleck, Sebastian, 22587 Hamburg (DE); Guhde, Ingo, 22587 Hamburg (DE); Rehberg, Michaela, 22587 Hamburg (DE)
(74) Vertreter: Uexküll & Stolberg

(56) Entgegenhaltungen:
- GB-A- 2 171 408
- US-A1- 2009 162 440

## Beschreibung

Die Erfindung betrifft eine gefriergetrocknete pharmazeutische Zusammensetzung mit Gehalt an Mitomycin C, die sich durch eine hohe Stabilität und Reinheit auszeichnet und schnell zu gebrauchsfertigen Lösungen rekonstituiert werden kann.

Mitomycin C, chemischer Name (laS,8S,8aR,8bS)-6-Amino-8-[[(aminocarbonyl)oxy]methyl]-1,1a,2,8,8a,8b-hexahydro-8a-methoxy-5-methyl-azirino[2',3':3,4]pyrrolo[1,2-a]indol-4,7-dion oder 6-Amino-8a-methoxy-5-methyl-4,7-dioxo-1,1a,2,4,7,8,8a,8b-octahydroazarino[2',3':3,4]pyrrolo[1,2-a]indol-8-ylmethyl carbamat, wurde erstmals 1958 aus Streptomyces caespitosus isoliert (siehe Wakaki et al, Antibiotics and Chemotherapy, 8, 228, 1958 und GB 830,874). Es hat eine ausgezeichnete Antitumor-Wirkung und kann vor allem zur Behandlung von Blasentumoren, Magenkarzinomen, Bronchialkarzinomen, Pankreaskarzinomen, Kolon-Rektum-Karzinomen, Mammakarzinomen, Leberzellkarzinomen, Zervixkarzinomen, Sophaguskarzinomen, Karzinomen im Kopf-HalsBereich, chronischer myeoloischer Leukämie und dem Osteosarkom angewendet werden. Es wird dabei in Form von wässrigen Injektions- oder Infusionslösungen oder Lösungen zur intravesikalen Anwendung eingesetzt.

Die US 5,216,011 beschreibt Lösungen von Mitomycin C, die z.B. durch Injektion verabreicht werden können. Mit diesen Lösungen sollen Schwierigkeiten bei der Herstellung von gefriergetrockneten Präparaten umgangen werden. Die Lösungen enthalten als Lösungsmittel 40 bis 100 Vol.-% Propylenglykol und 0 bis 60 Vol.-% Wasser.

Die US 2009/0162440 beschreibt gefriergetrocknete Mitomycin-haltige Mikrokügelchen.

Weiter ist aus der DE 199 57 371 eine wässrige Lösung von Mitomycin C bekannt, die Puffer enthält und einen pH-Wert im Bereich von 6,0 bis 9,0 aufweist. Darüber hinaus zeichnet sich die Lösung durch ein spezielles Verhältnis von Konzentration an Mitomycin C zu Ionenstärke des Puffers aus.

Allerdings ist die Haltbarkeit von gebrauchsfertigen Lösungen von Mitomycin nicht ausreichend, da sich der Wirkstoffgehalt bei der Lagerung stark verringert. Aus diesem Grund wird Mitomycin C in Form von Lyophilisaten oder Trockenpulvern hergestellt, und diese Darreichungsformen werden unmittelbar vor der Verabreichung mit sterilem Lösungsmittel rekonstituiert, um gebrauchsfertige Lösungen zu ergeben.

Lyophilisate werden z.B. unter der Marke "Mitem" vertrieben. Trockenpulver, z.B. in Mischung mit NaCl, sind z.B. unter der Marke "Mito-medac" erhältlich.

Die auf dem Markt befindlichen Lyophilisate haben jedoch den Nachteil, dass für sie nur eine relativ kurze Haltbarkeitsdauer von maximal zwei Jahren angegeben wird. Dies liegt an der hohen Instabilität des Mitomycin C, das sich in der Lyophilisierungslösung leicht unter Bildung einer Reihe von Abbauprodukten zersetzt. Bei den Abbauprodukten handelt es sich vor allem um Albomitomycin C, D1 und D2, deren Strukturformeln zusammen mit der von Mitomycin C nachstehend wiedergegeben sind.

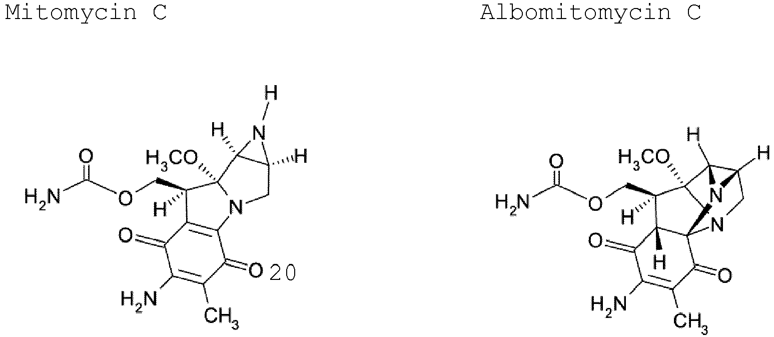

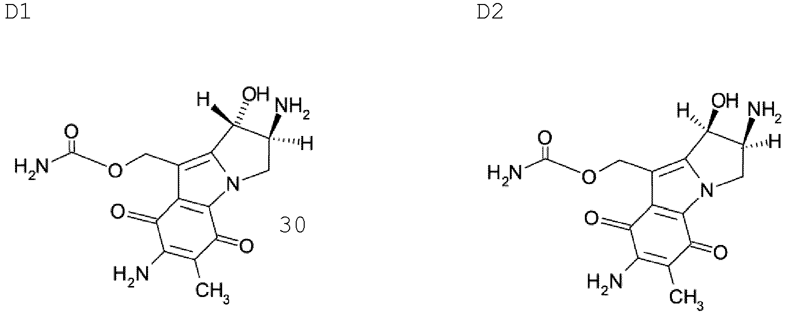

Trockenpulver von Mitomycin C haben zwar eine längere Haltbarkeit von ca. vier Jahren, sie haben aber den Nachteil, dass bei ihrer Herstellung Stäube entstehen, die zu einer gesundheitsgefährdenden Kontamination des Personals und Kontamination der Produktionsanlagen führen. Der erforderliche lange Zeitraum zur vollständigen Auflösung dieser Trockenpulver ist ebenfalls nicht zufriedenstellend.

Bei den bekannten Lyophilisaten und Trockenpulvern erfolgt die Rekonstitution zu Lösungen üblicherweise auf Endkonzentrationen von 1 mg/ml Mitomycin C, z.B. 20 mg Mitomycin C auf 20 ml Wasser oder isotonische Kochsalzlösung (0,9% Gew.-%). Bekannt sind auch niedrigere Konzentrationen nach Verdünnung auf 0,2 und 0,15 mg/ml für ophthalmologische Anwendungen. Eine höhere Endkonzentration in Wasser ist aufgrund der schlechten Löslichkeit der bekannten Produkte jedoch nicht möglich. Deren Sättigungskonzentration wird in der Literatur mit 0.5-1.0 mg/ml angegeben.

Der Erfindung liegt somit die Aufgabe zugrunde, die Nachteile der bekannten Lyophilisate und Trockenpulver zu vermeiden und insbesondere ein Verfahren zur Herstellung einer gefriergetrockneten pharmazeutischen Zusammensetzung von Mitomycin C zur Verfügung zu stellen, dessen Durchführung zu keiner wesentlichen Reduzierung des Wirkstoffgehaltes führt und das zu einer Zusammensetzung führt, die eine hohe Stabilität und Reinheit aufweist und daher auch bei längerer Lagerung nur einen geringen Abbau des Wirkstoffs zeigt. Darüber hinaus soll die erzeugte pharmazeutische Zusammensetzung schnell und vollständig zu gebrauchsfertigen Lösungen rekonstituierbar sein, die eine hohe Konzentration an Wirkstoff aufweisen können.

Diese Aufgabe wird durch die gefriergetrocknete pharmazeutische Zusammensetzung nach den Ansprüchen 1 bis 8 gelöst.

Die erfindungsgemäße gefriergetrocknete pharmazeutische Zusammensetzung mit Gehalt an Mitomycin C zeichnet sich dadurch aus, dass sie Harnstoff enthält und durch ein Verfahren erhältlich ist, bei dem eine Lösung von Mitomycin C gefriergetrocknet wird, wobei die Lösung mindestens ein organisches Lösungsmittel enthält.

Die in dem Verfahren der Gefriertrocknung unterworfene Lösung wird im Folgenden auch als "Lyophilisierungslösung" bezeichnet.

Das in dem Verfahren verwendete organische Lösungsmittel ist insbesondere aus der Gruppe aus tert.-Butanol, n-Propanol, n-Butanol, Isopropanol, Ethanol, Methanol, 1-Pentanol, Chlorbutanol, Essigsäure, Aceton, Dimethylcarbonat, Acetonitril, Dichlormethan, Methylethylketon, Methylisobutylketon, Methylacetat, Tetrachlorkohlenstoff, Dimethylsulfoxid, N,N-Dimethylacetamid, Hexafluoraceton, Dimethylsulfon und Cyclohexan ausgewählt. Bevorzugt ist das organische Lösungsmittel aus der Gruppe aus tert.-Butanol, Ethanol, Isopropanol, Aceton, Dimethylsulfoxid und N,N-Dimethylacetamid ausgewählt. Besonders bevorzugt ist das organische Lösungsmittel tert.-Butanol.

In einer Ausführungsform stellt das organische Lösungsmittel das einzige Lösungsmittel in der Lösung dar. Bei Verwendung von organischen Lösungsmitteln, die bei Raumtemperatur fest sind, wird das Mitomycin C üblicherweise in vorher geschmolzenem organischen Lösungsmittel aufgelöst.

In einer weiteren, bevorzugten Ausführungsform enthält die in dem Verfahren eingesetzte Lösung außerdem Wasser.

Die Auflösung von Mitomycin C in mindestens einem organischen Lösungsmittel, vorzugsweise in einem Gemisch mit Wasser, führt überraschenderweise zu einer Lyophilisierungslösung, die bei Raumtemperatur bis zu 48 h und bei 5°C sogar bis zu 9 Tage physikalisch und chemisch stabil ist. Damit bleibt auch der Gehalt an Verunreinigungen in dieser Lösung überraschend niedrig und somit die Reinheit der Lösung sehr hoch. Aufgrund der hohen Stabilität und der hohen Reinheit der Lösung können mit dem Verfahren sehr reine gefriergetrocknete Zusammensetzungen von Mitomycin C hergestellt werden.

Die Gefriertrocknung dieser Lösung führt überraschenderweise zu keiner wesentlichen Zunahme von Verunreinigungen, so dass auch die erhaltene gefriergetrocknete Zusammensetzung eine hohe Reinheit aufweist. Die Zusammensetzung zeigt überraschenderweise ebenfalls eine sehr hohe Stabilität, und sie weist auch nach Lagerung über mehrere Monate einen hohen Gehalt an Wirkstoff und nur geringe Mengen an unerwünschten Abbauprodukten auf.

Weiter zeigt die mit dem Verfahren erzeugte Zusammensetzung, im Folgenden auch als "Lyophilisat" bezeichnet, eine überraschend hohe Löslichkeit in üblichen zur Rekonstitution verwendeten Lösungsmitteln und ermöglicht die Herstellung von gebrauchsfertigen Lösungen, die Mitomycin C in einer Konzentrationen von mehr als 1 mg/ml enthalten. Durch Verwendung der Zusammensetzung können daher gebrauchsfertige Lösungen mit einer besonders hohen Wirkstoffkonzentration erzeugt werden.

Die im Verfahren eingesetzte Lösung enthält vorzugsweise ein Gemisch aus Ethanol und Wasser, ein Gemisch aus Isopropanol und Wasser, ein Gemisch aus Aceton und Wasser, ein Gemisch aus Dimethylsulfoxid und Wasser, oder ein Gemisch aus Dimethylsulfoxid und Dimethylacetamid. Besonders bevorzugt enthält die Lösung ein Gemisch aus tert.-Butanol und Wasser.

Das eingesetzte Gemisch aus Ethanol und Wasser enthält insbesondere mindestens 1 Gew.-%, bevorzugt mindestens 5 Gew.-%, besonders bevorzugt mindestens 10 Gew.-% und ganz besonders bevorzugt mindestens 15 Gew.-% Ethanol.

Das eingesetzte Gemisch aus Isopropanol und Wasser enthält insbesondere mindestens 1 Gew.-%, bevorzugt mindestens 5 Gew.-%, besonders bevorzugt mindestens 10 Gew.-% und ganz besonders bevorzugt mindestens 15 Gew.-% Isopropanol.

Das eingesetzte Gemisch aus Aceton und Wasser enthält insbesondere mindestens 1 Gew.-%, bevorzugt mindestens 5 Gew.-%, besonders bevorzugt mindestens 10 Gew.-% und ganz besonders bevorzugt mindestens 15 Gew.-% Aceton.

Das eingesetzte Gemisch aus Dimethylsulfoxid und Wasser enthält insbesondere mindestens 1 Gew.-%, bevorzugt mindestens 30 Gew.- %, besonders bevorzugt mindestens 50 Gew.-% und ganz besonders bevorzugt mindestens 80 Gew.-% Dimethylsulfoxid.

Das eingesetzte Gemisch aus Dimethylsulfoxid und Dimethylacetamid enthält insbesondere mindestens 5 Gew.-% und bevorzugt mindestens 50 Gew.-% Dimethylsulfoxid.

In dem Verfahren wird besonders bevorzugt eine Lösung eingesetzt, die ein Gemisch aus tert.-Butanol und Wasser enthält. Dieses Gemisch aus tert.-Butanol und Wasser enthält insbesondere mindestens 1 Gew.-%, bevorzugt mindestens 5 Gew.-%, besonders bevorzugt mindestens 10 Gew.-% und weiter bevorzugt mindestens 15 Gew.-% tert.-Butanol.

In einer ganz besonders bevorzugten Ausführungsform enthält das eingesetzte Gemisch aus tert.-Butanol und Wasser 80 bis 99 Gew.- %, insbesondere 84 bis 95 Gew.-%, bevorzugt 88 bis 92 Gew.-% und ganz besonders bevorzugt etwa 89 Gew.-% tert.-Butanol. In einer alternativen ganz besonders bevorzugten Ausführungsform enthält das eingesetzte Gemisch aus tert.-Butanol und Wasser 10 bis 30 Gew.-%, insbesondere 15 bis 25 Gew.-%, bevorzugt 18 bis 22 % und ganz besonders bevorzugt etwa 20 Gew.-% tert.-Butanol.

Die im Verfahren eingesetzte Lyophilisierungs-lösung enthält Mitomycin C üblicherweise in einer Konzentration von 0,1 bis 500 mg/g, insbesondere 0,5 bis 10,0 mg/g, bevorzugt 0,8 bis 6,0 mg/g und besonders bevorzugt 1,0 bis 4,0 mg/g.

Die Lösung enthält außerdem Harnstoff. Mit Hilfe dieses Zusatzes können Lyophilisate hergestellt werden, die eine besonders hohe Löslichkeit in zur Rekonstitution verwendeten Lösungsmitteln haben. Die Verwendung dieses gegenüber dem Wirkstoff inerten Hilfsstoffs führt zu einem Lyophilisat, das aufgrund seiner besonderen Eigenschaften den Wirkstoff vor destabilisierenden Einflüssen schützt und somit den Wirkstoff in seiner reinen Form bewahrt.

In einer bevorzugten Ausführungsform des Verfahrens enthält die Lösung Harnstoff in einer Konzentration von 1 bis 200 mg/g, insbesondere 5 bis 100 mg/g und bevorzugt 10 bis 60 mg/g.

Die Lösung kann ferner Polyethylenglykol oder Mannit enthalten.

Das Polyethylenglykol hat insbesondere ein mittleres Molekulargewicht (Zahlenmittel) von 1000 bis 8000 und bevorzugt von 2000 bis 8000. Besonders bevorzugt wird PEG 4000 als Polyethylenglykol eingesetzt, das ein mittleres Molekulargewicht (Zahlenmittel) von etwa 4000 aufweist.

In einer bevorzugten Ausführungsform des Verfahrens enthält die Lösung Polyethylenglykol in einer Konzentration von 1 bis 200 mg/g, insbesondere 5 bis 100 mg/g und bevorzugt 10 bis 60 mg/g.

In einer bevorzugten Ausführungsform des Verfahrens enthält die Lösung Mannit in einer Konzentration von 1 bis 200 mg/g, insbesondere 5 bis 100 mg/g und bevorzugt 10 bis 60 mg/g.

Die Lösung kann darüber hinaus auch noch andere übliche Hilfsstoffe wie weitere Zuckeralkohole, z.B. Isomalt, Lactit, Sorbit, Lactit, Sorbit, Xylit, Threit, Erythrit oder Arabit, Zucker, z.B. Saccharose, Glucose, Fructose, Maltose, Rhamnose, Lactose oder Trehalose, Aminosäuren, z.B. L-Phenylalanin, L-Tryptophan, L-Prolin, L-Histidin, L-Glycin oder L-Arginin, Polymere, z.B. Polyvinylpyrrolidone, Polyvinylacetate, Stärkederivate, z.B. Cyclodextrine, Dextrosen, Amylopektine, Amylosen, Polysaccharide, z.B. Alginate, Pektine, Cellulosen, Mittel zur Einstellung der Isotonie, z.B. Natriumchlorid, Calciumchloriddihydrat, Natriumacetattrihydrat, Dinatriumhydrogenphosphatdihydrat, und/oder Mittel zur Einstellung des pH-Wertes, z.B. Trometamol, enthalten, und zwar üblicherweise in Konzentrationen von jeweils 1 bis 200, insbesondere 5 bis 100 und bevorzugt 10 bis 60 mg/g.

Die Gefriertrocknung der Lyophilisierungslösung erfolgt in der Regel unter Verwendung von für pharmazeutische Zwecke üblicherweise eingesetzten Gefriertrocknungsprozessen und Geräten. In der Regel wird die Lösung in ein geeignetes Gefäß abgefüllt, und das Gefäß wird in einen üblichen Gefriertrockner mit kühl- und heizbaren Stellflächen gestellt, auf denen die Lösung den verschiedenen Temperaturen des Gefriertrocknungsverfahrens ausgesetzt werden kann. Zur Herbeiführung der Trocknung wird die Lösung üblicherweise gefroren und einem verminderten Atmosphärendruck ausgesetzt. Dadurch kommt es in hohem Maße zur Sublimation von Lösungsmittel aus der gefrorenen Lösung, welches sich z.B. an dafür vorgesehenen kühleren Bereichen des Gefriertrockners niederschlägt. Es schließt sich dann regelmäßig noch eine Sekundärtrocknung bei höheren Temperaturen an. Nach Abschluss der Gefriertrocknung lässt man in der Regel das erhaltene Lyophilisat auf Raumtemperatur kommen und verschließt das verwendete Gefäß unter sterilen Bedingungen. Ein geeignetes Programm zur Lyophilisation kann eine Beladung des Gefäßes bei Raumtemperatur, ein Gefrieren bei -50°C bis -35°C bei Normaldruck, anschließende Erniedrigung des Atmosphärendruckes und danach Erhöhung der Temperatur um 25 bis 95°C zur Herbeiführung der Trocknung umfassen.

Die mit dem Verfahren erhaltene gefriergetrocknete Zusammensetzung verfügt offenbar auch über eine besondere und vorteilhafte Struktur, da für die vollständige Rekonstitution zu einer gebrauchsfertigen Lösung ein nur sehr kurzer Zeitraum von weniger als 60 Sekunden, insbesondere weniger als 30 Sekunden und bevorzugt 4 bis 20 Sekunden ausreicht. Das stellt einen wichtigen Vorteil dar, weil es damit Klinikpersonal möglich ist, gebrauchsfertige Lösungen unmittelbar vor der beabsichtigten Verabreichung an den Patienten frisch herzustellen, ohne lange Wartezeiten für eine vollständige Auflösung in Kauf nehmen zu müssen. Gleichfalls nimmt bei solch kurzen Rekonstitutionszeiten das Risiko ab, dass es zu unerwünschten Abbaureaktion des Wirkstoffs kommt.

Für die Rekonstitution der Zusammensetzung wird üblicherweise isotonische Kochsalzlösung oder Wasser für Injektionszwecke verwendet. Möglich sind auch andere pharmazeutisch vertretbare Lösungen zur Rekonstitution, wie z.B. Ringer-Lactat-Lösungen oder Phosphatpuffer.

Die erfindungsgemäße gefriergetrocknete pharmazeutische Zusammensetzung enthält Harnstoff. Eine derartige Zusammensetzung benötigt zur Rekonstitution geringere Mengen an Rekonstituierungsmittel und somit kann mit ihrer Hilfe eine gebrauchsfertige Lösung mit einer besonders hohen Konzentration an Mitomycin C erzeugt werden.

Die Zusammensetzung enthält Harnstoff üblicherweise in einer Konzentration von 0,2 bis 1, insbesondere 0,5 bis 0,99, bevorzugt 0,8 bis 0,95 und besonders bevorzugt 0,93 bis 0,94 g pro Gramm Zusammensetzung.

Die Zusammensetzung kann ferner Polyethylenglykol üblicherweise in einer Konzentration von 0,2 bis 1, insbesondere 0,5 bis 0,99, bevorzugt 0,8 bis 0,95 und besonders bevorzugt 0,93 bis 0,94 g pro Gramm Zusammensetzung enthalten.

Die Zusammensetzung kann ferner Mannit üblicherweise in einer Konzentration von 0,2 bis 1, insbesondere 0,5 bis 0,99, bevorzugt 0,8 bis 0,95 und besonders bevorzugt 0,93 bis 0,94 g pro Gramm Zusammensetzung enthalten.

Die erfindungsgemäße Zusammensetzung enthält die Verunreinigungen D1, D2 und Albomitomycin C in einer Gesamtmenge von insbesondere weniger als 2,0 %, bevorzugt weniger als 1,5 %, besonders bevorzugt weniger als 1,0 %, weiter bevorzugt weniger als 0,8 % und ganz besonders bevorzugt weniger als 0,6 %, wobei die Menge dieser Verunreinigungen gemäß der Verunreinigungsmethode nach Ph Eur 8.0 Mitomycin Monographie 01/2008: 1655 bestimmt wird. Diese Bestimmungsmethode wird in den Beispielen auch als Methode 4 bezeichnet.

Die Erfindung wird im Folgenden anhand von Beispielen näher erläutert.

### Beispiele (Beispiele gemäß der Ansprüche enthalten Harnstoff und sind durch ein Verfahren erhältlich, bei dem eine Lösung von Mitomycin C gefriertrocknet wird, wobei die Lösung mindestens ein organisches Lösungsmittel enthält)

### Beispiel 1 - Löslichkeit und Stabilität von Mitomycin C in verschiedenen Lösungsmitteln

Es wurden die Löslichkeit und Stabilität von Mitomycin C in den verschiedenen in Tabelle 1 angegebenen Lösungsmitteln und Lösungsmittel-Gemischen untersucht.

**Tabelle 1 "Verwendete Lösungsmittel/-Gemische"**

| Nr. | | CoSolvens Gew.-% | Wasser Gew.-% | Konzentration Mitomycin C [mg/g] | Konzentration Mannit [mg/g] |
|---|---|---|---|---|---|
| 1 | Wasser | - | 100 | 2,0 | 4,0 |
| 2 | tert.-Butanol | 32,6 | 67,4 | 2,1 | 4,1 |
| 3 | Ethanol | 16,4 | 83,6 | 2,2 | 4,4 |
| 4 | Isopropanol | 16,2 | 83,8 | 2,2 | 4,3 |
| 5 | Aceton | 7,1 | 92,9 | 2,0 | 4,1 |

Zu diesem Zweck wurden unter Verwendung dieser Gemische jeweils Lösungen hergestellt, die 2,0 bis 2,2 mg/g Mitomycin C und 4,0 bis 4,4 mg/g Mannit enthielten.

Nach 1,5 h Rührzeit bei Raumtemperatur und unter Lichtausschluss wurden die erhaltenen Mischungen unter Bestrahlung mit linear polarisiertem Licht auf Partikel untersucht. Das Ergebnis der Sichtprüfung ist in Tabelle 2 aufgeführt.

**Tabelle 2 "Sichtprüfung"**

| **Nr.** | **Lösungsmittel/- Gemisch Gew.-%** | **Beschreibung der Mischungen** |
|---|---|---|
| 1 | Wasser 100% | Dunkelblaue Suspension, nicht vollständig gelöst |
| 2 | tert.-Butanol 32,6% | Dunkelblaue klare Lösung, vollständig gelöst |
| 3 | Ethanol 16,4% | Dunkelblaue klare Lösung, vollständig gelöst |
| 4 | Isopropanol 16,2% | Dunkelblaue klare Lösung, vollständig gelöst |
| 5 | Aceton 7,1% | Dunkelblaue klare Lösung, vollständig gelöst |

Weiter wurde der Gehalt an Mitomycin C und Verunreinigungen in den erhaltenen Mischungen nach 24 Stunden Lagerung bei Raumtemperatur bestimmt. Die entsprechenden Ergebnisse sind in Tabelle 3 aufgeführt.

**Tabelle 3 "Gehalt an Mitomycin C und Verunreinigungen nach 24 h Lagerung bei Raumtemperatur"**

| **Nr.** | **LösungsmittelGemisch Gew.-%** | **Gehalt Mitomycin C [%]*** | **Gesamtgehalt an Verunreinigungen (D1, D2 und Albomitomycin C) [%]**** |
|---|---|---|---|
| 2 | tert.-Butanol 32,6% | 98,04 | 1,96 |
| 3 | Ethanol 16,4% | 94,74 | 5,26 |
| 4 | Isopropanol 16,2% | 95,69 | 4,30 |
| 5 | Aceton 7,1% | 93,99 | 6,01 |

| | | | |
|---|---|---|---|
| * Bestimmung nach Methode 1 (s. Tabelle 5) ** Bestimmung nach Methode 2 (s. Tabelle 5) | | | |

Die in Tabelle 3 aufgeführten Ergebnisse zeigen, dass das Mitomycin C in der erfindungsgemäß eingesetzten Lyophilisierungslösung in einem Gemisch aus tert.-Butanol und Wasser eine besonders hohe Stabilität hat.

Weiterhin wurden die erhaltenen Mischungen nach 24 h Lagerung unter Lichtausschluss bei (a) Raumtemperatur und (b) 2 bis 8°C erneut unter Bestrahlung mit linear polarisiertem Licht auf sichtbare Partikel untersucht. Die dabei erhaltenen Ergebnisse sind in der Tabelle 4 aufgeführt.

**Tabelle 4 "Sichtprüfung nach Lagerung für 24 h"**

| **Nr.** | **Lösungsmittel/- Gemisch Gew.-%** | **Lagerung bei Raumtemperatur** | **Lagerung bei 2 bis 8°C** |
|---|---|---|---|
| 1 | Wasser 100% | Dunkelblaue Suspension; dispergierte Partikel | Dunkelblaue Suspension |
| 2 | tert.-Butanol 32,6% | Dunkelblaue klare Lösung | Dunkelblaue klare Lösung |
| 3 | Ethanol 16,4% | Dunkelblaue klare Lösung; nadelartige Partikel | Dunkelblaue klare Lösung; nadelartige Partikel |
| 4 | Isopropanol 16,1% | Dunkelblaue klare Lösung; | Dunkelblaue klare Lösung; nadelartige Partikel |
| 5 | Aceton 7,1% | Dunkelblaue klare Lösung; nadelartige Partikel | Dunkelblaue klare Lösung; nadelartige Partikel |

Mit den erfindungsgemäß einsetzbaren Lösungsmittel-Gemischen wurden deutlich stabilere Lösungen als mit Wasser erhalten. Die mit dem Gemisch aus 32,6 Gew.-% tert.-Butanol und 67,4 Gew.-% Wasser erhaltene Lösung erwies sich am stabilsten, da in ihr auch nach 24 h sowohl bei Raumtemperatur als auch bei 2 bis 8°C keine Partikel sichtbar waren. Des Weiteren war in der tert-Butanol-Wasser-Mischung nach 24 Stunden Lagerung bei Raumtemperatur der Gehalt an Mitomycin C am höchsten und der Gehalt an Verunreinigungen am geringsten.

### Bestimmung des Gehaltes an Mitomycin C und an Verunreinigungen mittels Hochdruck-Flüssigchromatographie

Die Proben werden jeweils auf 0,5 mg/ml Mitomycin C mit N,N-Dimethylacetamid verdünnt. Die Referenzlösung enthält 0,5 mg/ml Mitomycin C in N,N-Dimethylacetamid.

**Tabelle 5 "Bedingungen der Hochdruck-Flüssigchromatographie"**

| | Methode 1 | Methode 2 |
|---|---|---|
| Detektion: | UV bei 365 nm | UV bei 254 nm |
| Mobile Phase: | Puffer/Methanol 62,5/37,5, Vol/Vol | Puffer/Methanol 75/25, Vol/Vol |
| | Puffer: | Puffer: |
| | 0,025 % Essigsäure in 0,02 M Ammoniumacetat | 0,025 % Essigsäure in 0,02 M Ammoniumacetat |
| Fließrate: | 1,4 ml/min | 2,6 ml/min |
| Säule: | YMC-Pack Phenyl 4,6 mm x 300 mm, 10 µm | |
| Temperatur: | 25°C | |
| Injektionsvolumen: | 10 µl | |

### Beispiel 2 - Löslichkeit und Stabilität von Mitomycin C in Dimethylsulfoxid und einem Gemisch aus Dimethylsulfoxid und N,N-Dimethylacetamid

Es wurden die Löslichkeit und Stabilität von Mitomycin C in den in Tabelle 6 angegebenen Lösungsmitteln, nämlich in Dimethylsulfoxid (DMSO) sowie einem Gemisch aus Dimethylsulfoxid (DMSO) und N,N-Dimethylacetamid (DMAA) untersucht.

**Tabelle 6 "DMSO oder DMSO/DMAA als Lösungsmittel/-Gemisch"**

| Nr. | Dimethylsulfoxid Gew.-% | N,N-Dimethylacetamid Gew.-% |
|---|---|---|
| 6 | 5,7 | 94,3 |
| 7 | 100 | - |

Zu diesem Zweck wurden unter Verwendung dieser Lösungsmittel jeweils Lösungen hergestellt, die für Nr. 6 (DMSO/DMAA) 0,53 mg/g Mitomycin C und 8,0 mg/g Harnstoff und für Nr. 7 (DMSO) 1,82 mg/g Mitomycin C und 27,3 mg/g Harnstoff enthielten. Die Lösungen wurden bei Raumtemperatur und unter Lichtausschluss über einen Zeitraum von bis zu 71,5 Stunden gelagert.

**Tabelle 7a "Gehalt von Mitomycin in DMSO/DMAA-Gemisch"**

| | Gehalt Mitomycin [%] | | | | | |
|---|---|---|---|---|---|---|
| DMSO/DMAA Gew.-% | t=0 | t=6,5h | t=14,5h | t=21h | t=42h | t=71,5h |
| 5,7/94,3¹ | 100 | 99,93 | 99,62 | 98,84 | 99,11 | 98,58 |

| | | | | | | |
|---|---|---|---|---|---|---|
| ¹Gehalt bestimmt mit Methode 3 | | | | | | |

**Tabelle 7b "Reinheit von Mitomycin in DMSO"**

| | Reinheit Mitomycin [%] | | | | | |
|---|---|---|---|---|---|---|
| DMSO Gew.-% | t=0 | t=4h | t=6,5h | t=13h | t=20h | t=26,5h |
| 100² | 100 | 99,31 | 99,32 | 99,19 | 99,36 | 99,32 |

| | | | | | | |
|---|---|---|---|---|---|---|
| ² Reinheit bestimmt mit Methode 4; Reinheit ist definiert als Fläche des Mitomycin-Peaks in Relation zu der Gesamtfläche aller Peaks im HPLC-Chromatogramm | | | | | | |

In beiden Lösungsmitteln erwies sich das Mitomycin C als sehr stabil. Die Verwendung von DMSO alleine führte zu einer besonders niedrigen Abnahme des ursprünglichen Gehaltes an Mitomycin C von 100 % auf 99,32 % innerhalb eines Zeitraumes von 26,5 Stunden.

Der Gehalt an Mitomycin C und damit die Stabilität der erhaltenen Lösungen wurde mit den Methoden 3 und 4 überprüft. Methode 3 entspricht der Gehaltsmethode nach Ph Eur 8.0 Mitomycin Monographie 01/2008: 1655. Methode 4 entspricht der Verunreinigungsmethode nach Ph Eur 8.0 Mitomycin Monographie 01/2008: 1655.

### Beispiel 3 - Löslichkeit und Stabilität von Mitomycin C in verschiedenen tert.-Butanol/Wasser-Gemischen

Es wurden die Löslichkeit und Stabilität von Mitomycin C in den verschiedenen in Tabelle 8 angegebenen Gemischen aus tert.-Butanol und Wasser untersucht.

Zu diesem Zweck wurden unter Verwendung dieser Gemische jeweils Lösungen hergestellt, deren Konzentration an Mitomycin C 2,1 mg/g für Gemisch Nr. 8, 2,2 mg/g für Gemisch Nr. 9 und 2,4 mg/g für Gemisch Nr. 10 betrug.

Diese Lösungen wurden 16 h unter Lichtausschluss bei (a) Raumtemperatur und (b) bei 2 bis 8°C gelagert und anschließend erneut unter Bestrahlung mit linear polarisiertem Licht auf sichtbare Partikel untersucht. Die dabei erhaltenen Ergebnisse sind ebenfalls in der Tabelle 8 aufgeführt.

**Tabelle 8 "Sichtprüfung nach Lagerung für 16 h"**

| **Nr.** | **LösungsmittelGemisch Gew.-%** | **Lagerung bei Raumtemperatur (a)** | **Lagerung bei 2 bis 8°C (b)** |
|---|---|---|---|
| 8 | tert.-Butanol 32,6% | Dunkelblaue klare Lösung | Dunkelblaue klare Lösung |
| 9 | tert.-Butanol 51,7% | Dunkelblaue klare Lösung | Dunkelblaue klare Lösung |
| 10 | tert.-Butanol 72,9% | Dunkelblaue klare Lösung | Dunkelblaue klare Lösung |

Alle Gemische aus tert.-Butanol und Wasser waren in der Lage, die vorgegebene Menge an Mitomycin C aufzulösen, und in den erhaltenen Lösungen waren selbst nach 16 h Lagerung keine Partikel sichtbar.

Zur HPLC-Bestimmung der Stabilität von Mitomycin C in tert. Butanol/Wasser-Gemischen wurde zu einem Gemisch aus 20 Gew.-% tert.-Butanol und 80 Gew.-% Wasser (Nr. 11) Mitomycin C in einer Menge gegeben, um eine Lösung mit einer Konzentration von 10 mg/g Mitomycin C zu erhalten. Weiter wurde zu einem Gemisch aus 32,6 Gew.-% tert.-Butanol und 67,4 Gew.-% Wasser (Nr. 12) Mitomycin C in einer Menge gegeben, um eine Lösung mit einer Konzentration von 2,1 mg/g Mitomycin C zu erhalten. Weiter wurde zu einem Gemisch aus 89 Gew.-% tert.-Butanol und 11 Gew.-% Wasser (Nr. 13) Mitomycin C in einer Menge gegeben, um eine Lösung mit einer Konzentration von 5 mg/g Mitomycin C zu erhalten Weiter wurden eine Mitomycinhaltige Lösung in reinem tert.-Butanol (100 Gew.-%) (Nr. 14) und zum Vergleich eine Lösung in Wasser (Nr. 15) hergestellt. Bei dem Versuch mit reinem tert.-Butanol wurde dieses zunächst durch Erwärmen auf ca. 30°C geschmolzen und danach das Mitomycin C in der Schmelze aufgelöst.

Die Lösungen wurden dann 24 h bei Raumtemperatur in Braungläsern gelagert. Die entsprechenden Ergebnisse sind in Tabelle 9 aufgeführt.

**Tabelle 9 "Stabilität von Mitomycin C in tert.-Butanol/Wasser-Gemischen"**

| **Nr.** | **LösungsmittelGemisch Gew.-%** | **Mitomycin [mg/g]** | **Mitomycin Reinheit* t = 0h [%]** | **Mitomycin Reinheit* t = 6h [%]** | **Mitomycin Reinheit* t = 24h [%]** |
|---|---|---|---|---|---|
| 11 | tert.-Butanol 20% | 10 | 99,51 | 97,01 | 93,55 |
| 12 | tert.-Butanol 32,6% | 2,1 | 100,0 | 99,4 | 98,7 |
| 13 | tert.-Butanol 89% | 5 | 99,56 | 98,95 | 98,47 |
| 14 | tert.-Butanol 100% | 0,38 | 100,0 | 100,0 | 100,0 |
| 15 | Wasser (Referenz) | 0,48 | 96,8 | 92,1 | 89,9 |

| | | | | | |
|---|---|---|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | | | | |

In den tert.-Butanol-haltigen Lösungen hatte das Mitomycin C eine hohe Stabilität, wobei die Verwendung des Gemisches Nr. 12 und Nr. 13 zu einer besonders geringen Abnahme der ursprünglichen Reinheit bei t=0 von Mitomycin C von 100,0% auf 98,7% und 99,56% auf 98,47% führte. Eine besonders hohe Reinheit von 100,0 Gew.-% nach 24 h konnte in 100 Gew.-% tert.-Butanol erreicht werden, allerdings ist die Löslichkeit von Mitomycin C in reinem tert.-Butanol geringer.

Demgegenüber ist die Reinheit von Mitomycin C in Wasser nach 24h signifikant schlechter (bei t=0 von 96,8% auf 89,9%).

### Beispiel 4 - Kompatibilität von weiteren Hilfsstoffen mit tert. Butanol/Wasser Gemischen

Es wurden mehrere Lösungen hergestellt, indem jeweils 20 mg Mitomycin C in 6 g eines Gemisches aus 89 Gew.-% tert. Butanol und 11 Gew.-% Wasser aufgelöst wurden. Zu diesen Lösungen wurde die Hilfsstoffe Harnstoff, PEG 4000 oder Trometamol zugegeben, um jeweils eine Konzentration von 50 mg/g zu ergeben. Die Lösung mit Trometamol wurde außerdem mit Essigsäure auf einen pH-Wert von 7,4 eingestellt.

Bei t=0 sowie nach 6 h und 24 h bei Raumtemperatur wurden die Lösungen auf ihren Gehalt an Mitomycin C untersucht und mit einer Lösung verglichen, die keinen weiteren Hilfsstoff enthielt. Die Bestimmung des Gehaltes erfolgte gemäß Methode 2, und die Ergebnisse sind in Tabelle 10 aufgeführt.

**Tabelle 10 "Stabilität von Lösungen mit Harnstoff, Polyethylenglykol und Trometamol"**

| Hilfsstoff | Reinheit* Mitomycin C t=0 | Reinheit* Mitomycin C t=6 h | Reinheit* Mitomycin C t=24 h |
|---|---|---|---|
| Harnstoff | 99,69 | 99,76 | 99,14 |
| PEG 4000 | 100 | 99,58 | 99,50 |
| Trometamol | 99,56 | 99,44 | 99,50 |
| Ohne Hilfsstoff | 99,61 | 99,63 | 99,48 |

| | | | |
|---|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | | |

### Beispiel 5 - Stabilität von Mitomycin C in Harnstoff-haltigen tert. Butanol/Wasser Gemischen

In einem Gemisch aus 89 Gew.-% tert.-Butanol und 11 Gew.-% Wasser wurde Harnstoff in einer Menge aufgelöst, um eine Konzentration von 5 Gew.-% zu erzielen. Anschließend wurde Mitomycin C in einer Menge zugegeben, um eine Konzentration von 3,33 mg/g zu erhaltenen, und die Lösung wurde für eine Stunde gerührt. Die Lösung wurde in Braungläser abgefüllt und über 9 Tage bei Raumtemperatur (RT) ohne Lichtschutz, bei Raumtemperatur (RT) unter Lichtausschluss und bei 5°C unter Lichtausschluss gelagert.

Zu unterschiedlichen Zeitpunkten wurden Reinheitsbestimmungen gemäß Methode 2 durchgeführt. Die Ergebnisse sind in Tabelle 11 aufgeführt.

**Tabelle 11 "Stabilität von Mitomycin-Lösungen mit Harnstoff"**

| | Mitomycin Reinheit* [%] | | | | | |
|---|---|---|---|---|---|---|
| Lagerung | t=0 | t=6h | t=24h | t=48h | t=72h | t=9d |
| RT; ohne Lichtschutz | 99,32 | 98,83 | 97,60 | 96,77 | 96,14 | 95,78 |
| RT; unter Lichtausschluss | 99,33 | 98,83 | 97,80 | 97,04 | 96,74 | 96,35 |
| 5°C; unter Lichtausschluss | 99,23 | 99,18 | 99,20 | 98,86 | 98,80 | 98,21 |

| | | | | | | |
|---|---|---|---|---|---|---|
| * HPLC Bestimmung nach Methode 2; Reinheit ist definiert als Fläche des Mitomycin Peaks in Relation zu der Gesamtfläche aller Peaks im HPLC-Chromatogramm | | | | | | |

Selbst nach neun Tagen Lagerung war die Lösung unter allen Bedingungen noch überraschend stabil und die Reinheit der Lösung überraschend hoch.

### Beispiel 6 - Gefriertrocknung von Lösungen von Mitomycin C in verschiedenen tert.-Butanol/Wasser-Gemischen

Es wurden zunächst Lösungen von Mitomycin C in Gemischen aus tert.-Butanol und Wasser mit 16,1 Gew.-%, 32,6 Gew.-% und 72,9 Gew.-% tert.-Butanol hergestellt. Diese Lösungen enthielten 2,1 mg/g Mitomycin C und 4,1 mg/g Mannit für die Lösung mit dem Gemisch mit 16,1 Gew.-% tert.-Butanol, 2,2 mg/g Mitomycin C und 4,3 mg/g Mannit für die Lösung mit dem Gemisch mit 32,6 Gew.-% tert.-Butanol und 2,4 mg/g Mitomycin C und 4,8 mg/g Mannit für die Lösung mit dem Gemisch mit 72,9 Gew.-% tert.-Butanol.

Alle Lösungen waren klar und ausreichend stabil.

Zum Vergleich wurde ebenfalls eine Lösung von Mitomycin C in Wasser hergestellt, die 7 mg/g Mannit und wegen der schlechten Löslichkeit in Wasser 0,7 mg/g Mitomycin C enthielt.

Mittels konventioneller Gefriertrocknung mit (i) Einfrieren der Lösungen, (ii) Anlegen eines Vakuums, (iii) primärer und (iv) sekundärer Trocknung wurden Lyophilisate erhalten. Diese wurden mit Wasser rekonstituiert, und die erhaltenen rekonstituierten Lösungen wurden auf ihren Gehalt an Mitomycin C sowie von Verunreinigungen gemäß Beispiel 1 analysiert. Die Ergebnisse sind in der Tabelle 12 aufgeführt.

**Tabelle 12 "Gehalt an Mitomycin C und Verunreinigungen nach Gefriertrocknung und Rekonstitution"**

| Menge an tert. Butanol im Gemisch Gew.-% | Gehalt** Mitomycin C [%] | Gehalt*** Verunreinigungen [%] Gesamt; D1; D2; Albomitomycin C |
|---|---|---|
| tert.-Butanol 0% | 98,36 | 1,64; 0,40; 0,48; 0,76 |
| tert.-Butanol 16,1% | 99,55 | 0,46; NN*; 0,15; 0,31 |
| tert.-Butanol 32,6% | 99,81 | 0,20; NN*; 0,10; 0,10 |
| tert.-Butanol 72,9% | 100,00 | NN*; NN*; NN*; NN* |

| | | |
|---|---|---|
| * NN = Nicht nachweisbar, d.h. unterhalb der Nachweisgrenze ** HPLC Bestimmung nach Methode 1 *** HPLC Bestimmung nach Methode 2 | | |

Alle erfindungsgemäß eingesetzten Gemische aus tert.-Butanol und Wasser führten nach Lyophilisation und Rekonstitution zu Lösungen mit einem sehr hohen Gehalt an Mitomycin C und einem nur sehr geringen Gehalt an Verunreinigungen (D1, D2 und Albomitomycin C).

Demgegenüber ergab die Lyophilisation unter Verwendung von Wasser eine rekonstituierte Lösung mit deutlich geringerem Gehalt an Mitomycin C und einer sehr signifikanten Menge an Verunreinigungen.

### Beispiel 7 - Stabilität von Mitomycin C in tert.-Butanol-Wasser-Gemisch (89/11) mit 20 mg/g Harnstoff

Es wurden Lösungen von Mitomycin C in einem Gemisch aus 89 Gew.-% tert.-Butanol und 11 Gew.-% Wasser hergestellt. Die Lösungen enthielten neben 1,33 mg/g Mitomycin C ebenfalls jeweils 20 mg/g Harnstoff. Bei t=0 und nach 5, 22 und 27 Stunden bei Raumtemperatur (RT) unter Lichtausschluss und im Kühlschrank wurden die Lösungen auf ihre Reinheit gemäß Methode 2, untersucht. Die Ergebnisse sind in Tabelle 13 dargestellt.

**Tabelle 13 "Stabilität von Mitomycin C in Harnstoff-haltigen Lösungen 20 mg/g"**

| | Reinheit Mitomycin C* [%] | | |
|---|---|---|---|
| Bedingungen | t=5h | t=22h | t=27h |
| RT; unter Lichtausschluss | 99,7 | 99,2 | 98,9 |
| 5°C; unter Lichtausschluss | 99,8 | 99,8 | 99,7 |

| | | | |
|---|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | | |

Selbst nach mehr als einem Tag Lagerung bei Raumtemperatur ist die Reinheit der Lösung überraschend hoch, was die überraschend hohe Stabilität des Wirkstoffs in dem Lösungsmittel widerspiegelt.

### Beispiel 8 - Stabilität von Mitomycin C in tert.-Butanol-Wasser-Gemisch (89/11) mit 25 mg/g Harnstoff

Es wurden Lösungen von Mitomycin C in einem Gemisch aus 89 Gew.-% tert.-Butanol und 11 Gew.-% Wasser hergestellt. Die Lösungen enthielten neben 1,67 mg/g Mitomycin C ebenfalls jeweils 25 mg/g Harnstoff. Bei t=0 und nach 5, 22 und 27 Stunden bei Raumtemperatur unter Lichtausschluss und im Kühlschrank wurden die Lösungen auf ihre Reinheit gemäß Methode 2, untersucht.

**Tabelle 14 "Stabilität von Mitomycin C in Harnstoff-haltigen Lösungen 25 mg/g"**

| | Reinheit Mitomycin C * [%] | | |
|---|---|---|---|
| Bedingungen | t=5h | t=22h | t=27h |
| RT; unter Lichtausschluss | 99,6 | 99,1 | 98,6 |
| 5°C; unter Lichtausschluss | 99,8 | 99,5 | 99,5 |

| | | | |
|---|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | | |

Selbst nach mehr als einem Tag Lagerung bei Raumtemperatur ist die Reinheit der Lösung überraschend hoch, was die überraschend hohe Stabilität des Wirkstoffs in dem Lösungsmittel widerspiegelt.

### Beispiel 9 - Gefriertrocknung von Lösungen von Mitomycin C mit Gehalt an Harnstoff oder Polyethylenglykol (50 mg/g)

Es wurden Lösungen von Mitomycin C in einem Gemisch aus 89 Gew.-% tert.-Butanol und 11 Gew.-% Wasser hergestellt. Die Lösungen enthielten neben 3,33 mg/g Mitomycin C ebenfalls entweder jeweils 50 mg/g Harnstoff oder PEG 4000. Jeweils 6g dieser Lösungen wurden in kleine Fläschchen abgefüllt und in konventioneller Weise gefriergetrocknet. Die Kuchen der Lyophilisate mit Harnstoff und PEG 4000 waren fest und einwandfrei.

Die Lyophilisate wurden unmittelbar nach ihrer Herstellung (t = 0) sowie nach Lagerung für 1 Monat (t = 1 Monat) bei 40°C auf die Reinheit von Mitomycin C analysiert. Die Ergebnisse sind in der Tabelle 15 aufgeführt.

**Tabelle 15 "Stabilität von Lyophilisaten mit Harnstoff und Polyethylenglykol"**

| **t = 0;** | | | |
|---|---|---|---|
| **Hilfsstoff** | **Mitomycin Reinheit * [%]** | **Rekonstitutionszeit** | |
| Harnstoff | 99,65 | << 30 sek | |
| PEG 4000 | 99,34 | << 30 sek | |

| **t = 1 Monat bei 40°C;** | | | |
|---|---|---|---|
| **Hilfsstoff** | **Mitomycin Reinheit * [%]** | **Rekonstitutionszeit** | |
| Harnstoff | 99,89 | << 30 sek | |
| PEG 4000 | 96,19 | << 30 sek | |

| | | | |
|---|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | | |

Die erzeugten Lyophilisate hatten selbst nach Lagerung bei einer erhöhten Temperatur von 40°C eine hohe Reinheit an Mitomycin C, was ihre sehr gute Stabilität widerspiegelt.

Außerdem konnten die Lyophilisate in sehr viel weniger als 30 s mit isotonischer Kochsalzlösung vollständig rekonstituiert werden.

### Beispiel 10 - Gefriertrocknung von Lösungen von Mitomycin C mit Gehalt an Harnstoff (20 mg/g)

Es wurden Lösungen von Mitomycin C in einem Gemisch aus 89 Gew.-% tert.-Butanol (TBA) und 11 Gew.-% Wasser hergestellt. Die Lösungen enthielten neben 1,33 mg/g Mitomycin C ebenfalls jeweils 20 mg/g Harnstoff. Jeweils 1,5 g dieser Lösung wurden in kleine Fläschchen abgefüllt und in konventioneller Weise gefriergetrocknet. Die Kuchen der Lyophilisate waren fest und einwandfrei.

Die Lyophilisate wurden unmittelbar nach ihrer Herstellung (t=0) auf die Reinheit von Mitomycin C analysiert. Außerdem wurde die Rekonstitutionszeit bestimmt. Die Rekonstitution erfolgte auf eine Endkonzentration von 1 mg/g Mitomycin C mit isotonischer Kochsalzlösung. Die Ergebnisse sind in der Tabelle 16 aufgeführt.

**Tabelle 16**

| | **"Merkmale Harnstoff-haltige Lyophilisate"** | |
|---|---|---|
| **t = 0** | | |
| **Hilfsstoff** | **Mitomycin C Reinheit* [%]** | **Rekonstitutionszeit** |
| Harnstoff | 99,5 | << 30 sek |

| | | |
|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | |

Die erzeugten Lyophilisate hatten eine hohe Reinheit von Mitomycin C, was die sehr gute Stabilität des Wirkstoffs während des Prozesses widerspiegelt.

Außerdem konnten die Lyophilisate in sehr viel weniger als 30 s mit isotonischer Kochsalzlösung vollständig rekonstituiert werden.

### Beispiel 11 - Gefriertrocknung von Lösungen von Mitomycin C mit Gehalt an Harnstoff (25 mg/g)

Es wurden Lösungen von Mitomycin C in einem Gemisch aus 89 Gew.-% tert.-Butanol (TBA) und 11 Gew.-% Wasser hergestellt. Die Lösungen enthielten neben 1,67 mg/g Mitomycin C ebenfalls jeweils 25 mg/g Harnstoff. Jeweils 1,2 g dieser Lösung wurden in kleine Fläschchen abgefüllt und in konventioneller Weise gefriergetrocknet. Die Kuchen der Lyophilisate mit Harnstoff waren fest und einwandfrei.

**Tabelle 17**

| | **"Harnstoff-haltige Lyophilisate"** | |
|---|---|---|
| **t = 0** | | |
| **Hilfsstoff** | **Mitomycin Reinheit [%]*** | **Rekonstitutionszeit** |
| Harnstoff | 99,5 | << 30 sek |

| | | |
|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | |

Die erzeugten Lyophilisate hatten eine hohe Reinheit von Mitomycin C, was die sehr gute Stabilität des Wirkstoffs während des Prozesses widerspiegelt. Außerdem konnten die Lyophilisate in sehr viel weniger als 30 s mit isotonischer Kochsalzlösung vollständig rekonstituiert werden.

### Beispiel 12 - Mitomycin C in tert.-Butanol/Wasser-Gemisch (95/5) mit Mannit (45 mg/g)

Es wurden Lösungen von Mitomycin C in einem Gemisch aus 95 Gew.-% tert.-Butanol (TBA) und 5 Gew.-% Wasser hergestellt. Die Lösungen enthielten neben 5 mg/g Mitomycin C ebenfalls jeweils 45 mg/g Mannit. Jeweils 4,0 g dieser Lösung wurden in kleine Fläschchen abgefüllt und in konventioneller Weise gefriergetrocknet. Die Kuchen der Lyophilisate waren fest und einwandfrei.

**Tabelle 18**

| | **"Mannit-haltige Lyophilisate"** | |
|---|---|---|
| **t = 0** | | |
| **Hilfsstoff** | **Mitomycin Reinheit* [%]*** | **Rekonstitutionszeit** |
| Mannit | 99,6 | << 30 sek |

| | | |
|---|---|---|
| * HPLC Bestimmung nach Methode 2; Reinheit ist definiert als Fläche des Mitomycin-Peaks in Relation zu der Gesamtfläche aller Peaks im HPLC-Chromatogramm | | |

Die erzeugten Lyophilisate hatten eine hohe Reinheit von Mitomycin C, was die sehr gute Stabilität des Wirkstoffs während des Prozesses widerspiegelt. Außerdem konnten die Lyophilisate in sehr viel weniger als 30 s mit isotonischer Kochsalzlösung vollständig rekonstituiert werden.

### Beispiel 13 - Stabilität von Mitomycin-Lyophilisaten

Es wurden Lösungen von Mitomycin C in einem Gemisch aus 89 Gew.-% tert.-Butanol (TBA) und 11 Gew.-% Wasser hergestellt. Die Lösungen enthielten neben 3,33 mg/g Mitomycin C ebenfalls jeweils 50 mg/g Harnstoff. Jeweils 6 [entspricht 20 mg Mitomycin C] oder 12 g [entspricht 40 mg Mitomycin C] dieser Lösungen wurden in kleine Fläschchen abgefüllt und in konventioneller Weise gefriergetrocknet. Die erhaltenen Lyophilisate wurden zu unterschiedlichen Zeitpunkten t=0, t=3 Monate und t=6 Monate bei 25°C und 60 % relativer Feuchte (r.F.) oder 40°C und 75 % relativer Feuchte untersucht.

Die Bestimmung des Gehaltes an Mitomycin C erfolgte nach Methode 3 und die des Gehaltes an Verunreinigungen nach Methode 4. Die Rekonstitution erfolgte mit 20 bzw. 40 ml isotonischer Kochsalzlösung auf eine Endkonzentration von 1 mg/ml.

**Tabelle 19**

| **"Stabilität bei 40°C und 75 % r.F. [20 mg Mitomycin C]"** | | | |
|---|---|---|---|
| | t=0 | t=3 Monate | t=6 Monate |
| Rekonstitutionszeit | 7 sec | 12 sec | 17 sec |
| Gehalt Mitomycin C* | 97,8% | 98,2% | 98,3% |
| Verunreinigung Albomitomycin C** | 0,29% | 0,20% | 0,26% |
| Summe aller Verunreinigungen** | 0,29% | 0,55% | 0,61% |

| | | | |
|---|---|---|---|
| * Bestimmung nach Methode 3 ** Bestimmung nach Methode 4 | | | |

**Tabelle 20**

| **"Stabilität bei 40°C und 75 % r.F. [40 mg Mitomycin C]"** | | | |
|---|---|---|---|
| | t=0 | t=3 Monate | t=6 Monate |
| Rekonstitutionszeit | 6 sec | 20 sec | 18 sec |
| Gehalt Mitomycin C* | 97,2% | 96,6% | 96,7% |
| Verunreinigung Albomitomycin C** | 0,32% | 0,22% | 0,21% |
| Summe aller Verunreinigungen** | 0,32 | 0,55 | 0,58 |

| | | | |
|---|---|---|---|
| * Bestimmung nach Methode 3 ** Bestimmung nach Methode 4 | | | |

Die erzeugten Lyophilisate enthielten auch nach 6 Monaten Lagerung bei erhöhter Temperatur von 40 °C und erhöhter Luftfeuchte von 75 % r.F. nur sehr geringe Mengen an Verunreinigungen. Außerdem konnten die Lyophilisate in sehr viel weniger als 30 s mit isotonischer Kochsalzlösung vollständig rekonstituiert werden.

### Beispiel 14 - Erhöhte Endkonzentration nach Rekonstitution

Es wurden Lösungen von Mitomycin C in einem Gemisch aus 89 Gew.-% tert.-Butanol und 11 Gew.-% Wasser hergestellt. Die Lösungen enthielten neben 3,33 mg/g Mitomycin C ebenfalls jeweils 50 mg/g Harnstoff. Jeweils 6 g [entspricht 20 mg Mitomycin C] dieser Lösungen wurden in kleine Fläschchen abgefüllt und in konventioneller Weise gefriergetrocknet. Die erhaltenen Lyophilisate wurden mit 20 ml oder 10 ml Wasser für Injektionszwecke rekonstituiert.

| Endkonzentration Mitomycin C | Gehalt* Mitomycin C [%] | Beschreibung der rekonstituierten Lösung |
|---|---|---|
| 1 mg/ml | 100,6 | Klar, frei von sichtbaren Partikeln |
| 2 mg/ml | 100,6 | Klar, frei von sichtbaren Partikeln |

| | | |
|---|---|---|
| * Bestimmung nach Methode 3 | | |

Die rekonstituierten Lösungen in Wasser für Injektionszwecke hatten einen hohen Gehalt an Mitomycin C. Die Lösungen waren klar und frei von sichtbaren Partikeln.

## Patentansprüche

1. Gefriergetrocknete pharmazeutische Zusammensetzung mit Gehalt an Mitomycin C, die Harnstoff enthält und durch ein Verfahren erhältlich ist, bei dem eine Lösung von Mitomycin C gefriergetrocknet wird, wobei die Lösung mindestens ein organisches Lösungsmittel enthält.

2. Zusammensetzung nach Anspruch 1, die mindestens einen Hilfsstoff ausgewählt aus der Gruppe aus Polyethylenglykol und Mannit enthält.

3. Zusammensetzung nach Anspruch 1 oder 2, die Harnstoff in einer Konzentration von 0,2 bis 1, insbesondere 0,5 bis 0,99, bevorzugt 0,8 bis 0,95 und besonders bevorzugt 0,93 bis 0,94 g pro Gramm Zusammensetzung enthält.

4. Zusammensetzung nach Anspruch 2 oder 3, die Polyethylenglykol in einer Konzentration von 0,2 bis 1, insbesondere 0,5 bis 0,99, bevorzugt 0,8 bis 0,95 und besonders bevorzugt 0,93 bis 0,94 g pro Gramm Zusammensetzung enthält.

5. Zusammensetzung nach einem der Ansprüche 2 bis 4, die Mannit in einer Konzentration von 0,2 bis 1, insbesondere 0,5 bis 0,99, bevorzugt 0,8 bis 0,95 und besonders bevorzugt 0,93 bis 0,94 g pro Gramm Zusammensetzung enthält.

6. Zusammensetzung nach einem der Ansprüche 2 bis 5, bei der das Polyethylenglykol ein mittleres Molekulargewicht (Zahlenmittel) von 1000 bis 8000 und bevorzugt von 2000 bis 8000 aufweist.

7. Zusammensetzung nach einem der Ansprüche 1 bis 6, die die Verunreinigungen D1, D2 und Albomitomycin C in einer Gesamtmenge von weniger als 2,0 %, bevorzugt weniger als 1,5 %, besonders bevorzugt weniger als 1,0 %, weiter bevorzugt weniger als 0,8 % und ganz besonders bevorzugt weniger als 0,6 % enthält, wobei die Menge dieser Verunreinigungen gemäß der Verunreinigungsmethode nach Ph Eur 8.0 Mitomycin Monographie 01/2008: 1655 bestimmt wird.

8. Zusammensetzung nach einem der Ansprüche 1 bis 7, wobei die Lösung außerdem Wasser enthält.

## Claims

1. Freeze-dried pharmaceutical composition comprising mitomycin C, which comprises urea and is obtainable by a process, in which a solution of mitomycin C is freeze-dried, wherein the solution comprises at least one organic solvent.

2. Composition according to claim 1, which comprises at least one additive selected from the group of polyethylene glycol and mannitol.

3. Composition according to claim 1 or 2, which comprises urea at a concentration of 0.2 to 1, in particular 0.5 to 0.99, preferably 0.8 to 0.95 and particularly preferably 0.93 to 0.94 g per gram of composition.

4. Composition according to claim 2 or 3, which comprises polyethylene glycol at a concentration of 0.2 to 1, in particular 0.5 to 0.99, preferably 0.8 to 0.95 and particularly preferably 0.93 to 0.94 g per gram of composition.

5. Composition according to any one of claims 2 to 4, which comprises mannitol at a concentration of 0.2 to 1, in particular 0.5 to 0.99, preferably 0.8 to 0.95 and particularly preferably 0.93 to 0.94 g per gram of composition.

6. Composition according to any one of claims 2 to 5, in which the polyethylene glycol has an average molecular weight (number average) of 1000 to 8000 and preferably 2000 to 8000.

7. Composition according to any one of claims 1 to 6, which comprises the impurities D1, D2 and albomitomycin C in a total amount of less than 2.0%, preferably less than 1.5%, in particular less than 1.0%, more preferably less than 0.8% and particularly preferably less than 0.6%, wherein the amount of these impurities is determined in accordance with the impurity method as per Ph Eur 8.0 mitomycin monograph 01/2008: 1655.

8. Composition according to any one of claims 1 to 7, in which the solution additionally comprises water.

## Revendications

1. Composition pharmaceutique lyophilisée comprenant de la mitomycine C, qui contient de l'urée et que l'on peut obtenir par un procédé dans lequel on lyophilise une solution de mitomycine C, laquelle solution contient au moins un solvant organique.

2. Composition conforme à la revendication 1, qui contient au moins un auxiliaire choisi dans l'ensemble formé par un polyéthylèneglycol et du mannitol.

3. Composition conforme à la revendication 1 ou 2, qui contient de l'urée en une concentration de 0,2 à 1, en particulier 0,5 à 0,99, de préférence 0,8 à 0,95, et mieux encore 0,93 à 0,94 gramme par gramme de composition.

4. Composition conforme à la revendication 2 ou 3, qui contient un polyéthylèneglycol en une concentration de 0,2 à 1, en particulier 0,5 à 0,99, de préférence 0,8 à 0,95, et mieux encore 0,93 à 0,94 gramme par gramme de composition.

5. Composition conforme à l'une des revendications 2 à 4, qui contient du mannitol en une concentration de 0,2 à 1, en particulier 0,5 à 0,99, de préférence 0,8 à 0,95, et mieux encore 0,93 à 0,94 gramme par gramme de composition.

6. Composition conforme à l'une des revendications 2 à 5, dans laquelle le polyéthylèneglycol présente une masse molaire moyenne en nombre de 1000 à 8000, et de préférence de 2000 à 8000.

7. Composition conforme à l'une des revendications 1 à 6, qui contient les impuretés D1, D2 et albomitomycine C en une proportion totale inférieure à 2,0 %, en particulier inférieure à 1,5 %, de préférence inférieure à 1,0 %, mieux encore inférieure à 0,8 % et surtout inférieure à 0,6 %, étant entendu que la proportion de ces impuretés est déterminée selon la méthode pour impuretés indiquée dans Ph. Eur. 8.0, Mitomycine, monographie 01/2008, 1655.

8. Composition conforme à l'une des revendications 1 à 7, pour laquelle la solution contient en outre de l'eau.
